# EUROPEAN PATENT APPLICATION

(11) **EP 1 880 680 A2**
(43) Date of publication of application: **23.01.2008**
(21) Application number: 07075936.0
(22) Date of filing: 11.09.2003
(51) Int. Cl.: A61B 17/00, A61B 17/02

(54) **Septal puncture device**

(30) Priority: 23.09.2002 US 412952 P
(62) Divisional of application: 03754499.6
(71) Applicant: NMT Medical, Inc., Boston, MA 02210 (US)
(72) Inventor: Chanduszko, Andrzej, South Benson MA, 02127 (US); Callaghan, David J., Boston MA 02125 (US); Widomski, David, Wakefield MA 01880 (US)
(74) Representative: Greenwood, John David

(57) **Abstract**

A surgical apparatus for engaging an intracardiac device with a tissue in a patient, comprising and elongate member comprising a lumen (110), a delivery member (120) disposed within the lumen of the elongate member, said delivery member (120) having a proximal end and a distal end, and a flexible coil member (142"") comprising a first end and a second end, said first end being connected to the distal end (124) of the delivery member (120), said flexible coil member (142"") transitionable between a first position wherein said flexible coil member (142"") is collapsed and substantially parallel to the longitudinal axis of the elongate member (104) within the lumen (110) of the elongate member (104), and a second position wherein said flexible coil member (142"") is expanded to form a spiral comprising a plurality of loops, at least one of said loops defining a plane that is non-parallel to the longitudinal axis of the elongate member when said second portion is extended from the lumen (110) of the elongate member (104).

## Description

### RELATED APPLICATIONS

This application claims the benefit of U.S. Provisional Patent Application Serial No. 60/412,952, filed on September 23, 2002, and entitled "Septal Puncture Device," the entire contents of which are incorporated by reference herein.

### FIELD OF THE INVENTION

The present invention relates generally to apparatus and methods for stabilizing and/or forming openings in tissues, for example, a system that enables a flexible member to position a cutting member relative to a tissue surface such that an incision can be made by the cutting member in the tissue and more particularly, to a septal puncture apparatus including a flexible member and a cutting member that positions the cutting member for introducing a hole in the atrial septum near a patent foramen ovale defect to aid in closure of the defect by implanting a prosthetic occlusion device in a patient or using a remote apparatus for joining tissue (e.g., a remote suturing, stapling, gluing, or tissue welding tool).

### BACKGROUND OF THE INVENTION

Defects in the septum or vessels of the heart take various forms. The defects sometimes exhibit themselves as occlusions in a vessel or as openings in a chamber of the heart, tissue, or vascular wall.

A septum is generally defined as a dividing wall, membrane, or tissue between two or more bodily spaces. A defect sometimes found in the wall of the heart involves an opening or fluid communication between two chambers of the heart, for example, between the atria. One defect is specifically referred to as a patent foramen ovale (PFO). A PFO is anatomically comprised of two layers of partially overlapping but unfused cardiac tissue, forming a tunnel-like hole or opening between the left and right atria. The existence of this defect can place the patient at a high risk of embolic stroke by allowing for blood in the heart to be abnormally shunted between the two atria.

Septal defects are often treated using septal occluders commonly described and shown in U.S. Pat. No. 5,451,235 to Lock, et al., U.S. Pat. No. 5,578,045 to Das, and U.S. Pat. No. 6,214,029 to Thill, et al., the entirety of which are incorporated by reference herein. The devices typically include a pair of occlusion members that are connected to one another. The occluders typically allow for the occlusion members to be positioned on opposing sides of a hole or opening thereby obstructing the flow of blood or plasma through the opening.

Due to the tunnel-like nature of many PFO's, however, occluders often do not sit flush with the septal wall when implanted directly through the defect opening. For this reason, the use of septal puncture has been proposed and clinical success demonstrated in C.E. Ruiz, E.T. Alboliras, S.G. Pophal, The Puncture Technique: A New Method For Transcatheter Closure of Patent Foramen Ovale, Catheter and Cardiovascular Intervention 2001,53,369-372 (2001). Septal puncture allows for the creation of an opening through the septal wall in a more desirable location to maximize the likelihood that an occluder will sit flush with the surface of the septal wall overlapping a septal defect, thereby occluding the defect.

A punch-type device for performing a septal puncture procedure is discussed in U.S. Pat. No. 5,403,338 to Milo. However, in septal punctures, the punch or needles used pose a high risk of inadvertent puncture or damage to tissue other than septum primum and is a significant disadvantage of this procedure. For PFO closure, this risk is potentially even higher, due to the fact that there is defective and often thinning septal tissue, which may stretch an even greater amount risking inadvertent puncture of an unintended anatomical structure or suffer trauma during the puncture procedure.

The present invention addresses these risks.

### SUMMARY OF THE INVENTION

The present invention relates to apparatus, systems, and methods for constraining the motion of biological material, such as tissue walls, adjacent vessels or adjacent regions of an organ and forming a hole in the biological material such that access to opposing surfaces of the biological material can be achieved.

It is another aspect of this invention to provide apparatus and methods for inserting and positioning an elongate member and at least one flexible member in the heart. It is another aspect of this invention to provide apparatus and methods for limiting the motion of the septal wall of a heart using the at least one flexible member coupled to a distal end of a delivery member. It is another aspect of this invention to cut a hole in the septal wall of the heart while the at least one flexible member limits motion of the septal wall. Subsequent to cutting a hole in the tissue this present invention also contemplates inserting an apparatus for obstructing holes in the septal wall to limit the flow of blood between adjacent sections of the heart.

In general, in one aspect, the invention involves an apparatus for forming a hole in a tissue in a patient. The apparatus includes an elongate member having a longitudinal axis and at least one flexible member. The at least one flexible member has a first and second end. The second end of the at least one flexible member is free and the first end is fixed to the first elongate member. The at least one flexible member is movable between a first contracted position and a second extended position. In the first contracted position the at least one flexible member substantially parallels the longitudinal axis of the first elongate member. In the second extended position the at least one flexible member is substantially planar in shape, the plane defines a plurality of axes that lie in the plane, and the plurality of axes are non-parallel to the longitudinal axis of the elongate member. The at least one flexible member is capable of transitioning between the first position and the second position. The at least one flexible member is sized and shaped for contact with a first side of the tissue in a patient when the flexible member is in the second extended position.

Embodiments of this and other aspects of the invention can include the following features. The shape of the flexible members can be polygonal, circular, and/or ellipsoidal. The flexible members can include a wire loop. The at least one flexible member can include a section for stiffening the flexible member. In the second extended position, at least one of the plurality of axes defines an angle relative to the longitudinal axis of the elongate member that is between about 0 degrees and about 180 degrees. The at least one flexible member can limit movement of the tissue when the at least one flexible member is in the second extended position. The flexible members can be biased relative to the first elongate member.

. A cutting member for producing a hole in the tissue can be included in alternative embodiments of this invention. The cutting member can produce a hole in the tissue as the at least one flexible member limits movement of the tissue. The cutting member can be disposed within a lumen of the first elongate member and can be axially movable within the lumen. The cutting member can cut the tissue upon emerging from an opening in the lumen. The cutting member could be a needle or sharpened wire. An occlusion device can be included in alternative embodiments of the invention for occluding holes in the tissue. The occlusion device can be a septal occluder, suture, staple, or adhesive. A tissue joining apparatus can be included. The tissue joining apparatus can be a tissue welding apparatus. A second elongate member having a lumen can be provided and the first elongate member can axially move the at least one flexible member substantially co-linearly with a first lumen of the second elongate member.

In general, in another aspect, the invention involves an apparatus that includes a first elongate member having at least a first lumen and a longitudinal axis. The apparatus also includes a plurality of flexible members each having a first free end and a second end fixed relative to each other. Each flexible member is movable between a first contracted position and a second extended position. In the first contracted position each flexible member substantially parallels the longitudinal axis of the first elongate member. In the second extended position, the plurality of flexible members are substantially planar in shape, the plane defines a plurality of axes lying in the plane, and the plurality of axes are non-parallel to the longitudinal axis of the first elongate member. At least one of the plurality of flexible members is in contact with at least a first surface of a tissue in a patient when the at least one flexible member is in the second extended position.

Embodiments of this and other aspects of the invention can include the following features. The shape of the flexible members can be polygonal, circular, and/or ellipsoidal. The apparatus can include a second elongate member that is coupled to at least one flexible member for axially moving the at least one flexible member substantially co-linearly with the first lumen.

In general, in another aspect, the invention relates to a method of stabilizing a tissue in the body of a patient. The method involves placing a first flexible member in contact with a first side of a tissue in a patient and placing a second flexible member in contact with a second side of the tissue in the patient. The method also involves applying pressure with at least one of the first and second flexible members to the tissue.

This method of stabilizing a tissue can further include providing a cutting member for forming a hole in the tissue. This method of forming a hole in a tissue can further include providing an occlusion device for occluding the hole in the tissue. This method can include providing a tissue joining apparatus for joining tissue.

In general, in another aspect, the invention relates to a method for stabilizing a tissue in the body of a patient. The method involves extending a plurality of flexible members from a first lumen of a first elongate member. The method also involves placing at least one of the flexible members in contact with at least a first surface of the tissue. The method also involves applying pressure with the at least one flexible member to the tissue.

In general, in another aspect, the invention involves an apparatus for producing a hole in a tissue in a patient. The apparatus includes a catheter having a first lumen that has an opening. The apparatus also includes a delivery member that is axially movable within the first lumen of the catheter. The delivery member has a first distal end that extends from the opening in the catheter and a second lumen that has an opening. The apparatus also includes a cutting member that is axially movable, within the second lumen of the delivery member. The cutting member has a second distal end that extends from the opening in the delivery member and a third lumen that has an opening.

The apparatus can include a guidewire that is axially movable within the third lumen of the cutting member. The guidewire has a third distal end that extends from the opening in the cutting member. The apparatus can include a flexible member that has at least a first free end and a second free end. The first free end and the second free end each undergo a first articulation and a second articulation.

In general, in another aspect, the invention involves an apparatus for producing a hole in a tissue in a patient. The apparatus includes an elongate member having a first lumen that has an opening. The apparatus also includes a coil member that has a first portion and a second portion and is axially movable within the lumen of the elongate member. The coil member is sized and shaped for being gradually transferred out of the opening in the elongate member for placement of the first portion of the coil member adjacent to a first side of a tissue in a patient, and for placement of the second portion of the coil member adjacent a second side of the tissue. Embodiments of the invention can include a cutting member that is axially movable within the lumen of the elongate member in which the cutting member has a distal end that extends from the opening in the elongate member.

The foregoing and other objects, aspects, features, and advantages of the invention will become more apparent from the following description and from the claims.

### BRIEF DESCRIPTION OF THE DRAWINGS

In the drawings, like reference characters generally refer to corresponding parts throughout the different views. The drawings are not necessarily to scale, emphasis instead generally being placed on illustrating the principles and concepts of the invention.

FIG. 1 is a fragmented illustration of a septal puncture apparatus according to an illustrative embodiment of the invention.

FIG. 2 is a schematic side view of a portion of a septal puncture apparatus including a set of flexible members according to an illustrative embodiment of the invention.

FIG. 3A is a schematic side view of a portion of an embodiment of a septal puncture apparatus including a set of flexible members partially extended from an elongate member according to the invention.

FIG. 3B is a schematic side view of the flexible members of FIG. 3A fully extended from the opening in the elongate member.

FIG. 4 is a schematic side view of another embodiment of a set of flexible members according to the invention.

FIG. 5A is a schematic side view of an embodiment of a flexible member according to the invention.

FIG. 5B is a schematic end-on view of the flexible member of FIG. 5A.

FIG. 6A is a schematic side view of an embodiment of a flexible member according to the invention.

FIG. 6B is a schematic end-on view of the flexible member of FIG. 6A.

FIG. 7A is a schematic side view of an embodiment of a set of flexible members, a cutting member, and an elongate member of a portion of a septal puncture apparatus according to the invention.

FIG. 7B is an illustration of the set of flexible members and the cutting member extended out of the elongate member of FIG. 7A.

FIG. 8 is a schematic side view of a portion of a septal puncture apparatus including an occlusion device disposed within a lumen of a delivery member according to an illustrative embodiment of the invention.

FIG. 9 is a partially broken-away view of a heart depicting a portion of a septal puncture apparatus, according to the invention, on a second side of the septal wall.

FIG. 10A is a cross-sectional view of a septal wall of a heart depicting a set of flexible members located outside an opening in an end of an elongate member, according to an illustrative embodiment of the invention.

- FIG.10B is a cross-sectional view of the flexible members of FIG. 10A in which a portion of the flexible members is located in contact with a first side of a septal wall and another portion of the flexible members is located in proximity to a second side of the septal wall.

FIG. 10C is a cross-sectional view of the flexible members of FIGS. 10A and 10B in which a cutting member is extended from a lumen in the delivery member creating a hole through the septal wall.

FIG. 11 is a schematic side view of a flexible member, a cutting member, and an elongate member according to an illustrative embodiment of the invention.

FIG. 12A is a cross-sectional view of an illustrative embodiment of a flexible member and elongate member of the invention.

FIG. 12B is a cross-sectional view of the elongate member of FIG. 12A depicting inserting an occlusion device into a hole in the septal wall.

FIG. 12C is a cross-sectional view of the occlusion device of FIG. 12B obstructing holes in the septal wall of a patient.

FIG. 13 is an illustration of an exemplary set of flexible members according to an illustrative embodiment of the invention located in proximity to a patent foramen ovale defect.

### DESCRIPTION OF THE INVENTION

The present invention relates to apparatus, systems, and methods for stabilizing (e.g., constraining) the motion of biological material, such as tissue walls, and forming a hole in the biological material. In general overview, a system may include an elongate member for positioning and deploying a flexible member for stabilizing tissue. In one embodiment, the system includes a plurality of flexible members, at least one flexible member positionable on a side of the tissue opposite to another flexible member. Optionally, the system may further include a cutting member for cutting a hole in the septal wall of a heart to position and deploy a prosthetic occlusion device to obstruct blood flow through a septal defect of a cardiac septal wall. Optionally, the system may include a tissue joining apparatus for joining tissue in the body of a patient.

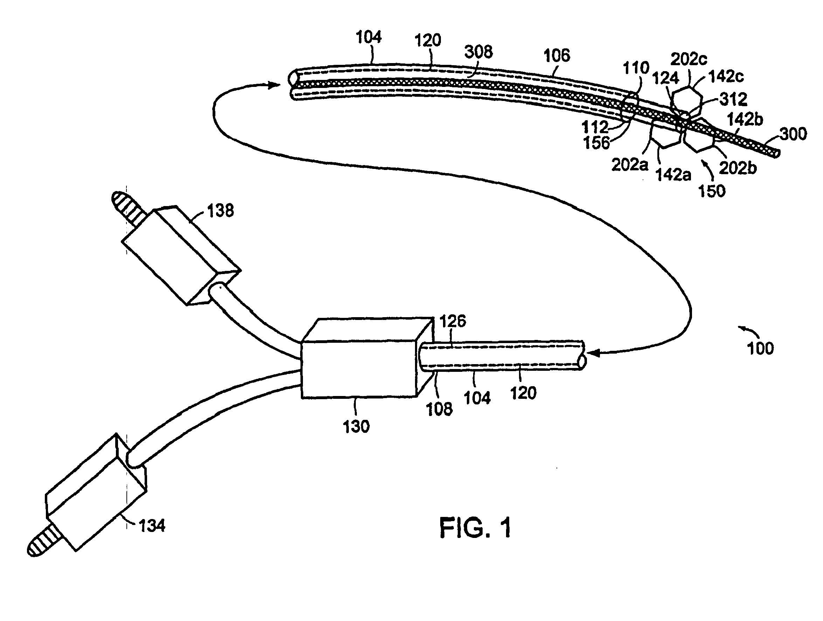

FIG. 1 illustrates a septal puncture apparatus 100 including three flexible members 142a, 142b, and 142c (generally 142) according to an illustrative embodiment of the invention. The illustrative flexible members 142a, 142b, and 142c couple to a delivery member 120 for applying, e.g., a pressure or force to a region in a body by pushing, pulling, or restraining the tissue, thereby stabilizing the tissue. In the illustrative embodiment, the flexible members 142a, 142b, and 142c are each hexagonal in shape and coupled to a distal end 124 of the delivery member 120, thereby forming, generally, a planar array 150. The plane of the array 150 defines a plurality of axes that lie in the plane. The plurality of axes are non-parallel to (i.e., biased relative to) the longitudinal axis of an elongate member 104. The normal to a plane represents a straight line extending away perpendicularly from the surface of the plane. The illustrative elongate member 104 is a tube. The delivery member 120 is slideably receivable within a lumen 110 of the elongate member 104. A distal end 106 of the elongate member 104 has an opening 112 and a rim 156. Instruments, e.g., the delivery member 120 and a cutting member 300, are slideably receivable in the lumen 110 of the elongate member 104. In this embodiment, the cutting member 300 is slideably receivable in a lumen 308 of the delivery member 120 and extends distally or withdraws proximally from an opening 312 at the distal end 124 of the delivery member 120.

FIG. 1 also illustrates an exemplary interface 130 that permits controllers, for example, a set of apparatus controllers 134 and 138 to communicate with the elongate member 104 and the delivery member 120, respectively. The exemplary controllers 134 and 138 extend, retract, or otherwise manipulate, e.g., the elongate member 104 and the delivery member 120, respectively. A single controller, could, alternatively, control all functions and operations of the tissue puncture apparatus 100 and the instruments disposed therein. Alternatively, a plurality of controllers (e.g., a distal end controller for extending the distal end of an instrument, a tip bending controller for altering the angular orientation of a portion of an instrument, and an extension controller for extending a component of an instrument) may be provided, each one controlling different components or functions of the septal puncture apparatus 100, as is known to one skilled in the art.

A proximal end 108 of the elongate member 104 and a proximal end 126 of the delivery member 120 operatively couple to the interface 130 thereby permitting the controllers 134 and 138 to communicate with the elongate member 104 and the delivery member 120, respectively. By way of example, the elongate member 104 and the delivery member 120 are flexible tubes fabricated from a biocompatible material, e.g., polyethylene, polyether-amide block co-polymer_(PEBAX^{™}),polyurethane, of fluorinated ethylene propylene.

By way of example, the elongate member 104 is a transeptal sheath, such as item number RCF-10.0-38-80-J-RB Large Check-Flo® Blue Introducer Set manufactured by Cook Incorporated of Bloomington, IN.

The three exemplary flexible members 142a, 142b, and 142c define the planar array 150, in the illustrative embodiment shown in FIG. 1. Each of the flexible members 142 include a first end (generally 204) which is joined to a distal end 124 of the delivery member 120. The first ends join to the distal end 124 by, for example, adhesive, structural epoxy, a thermal bond, or a mechanical fastener. The flexible members 142a, 142b, and 142c, alternatively, attach to the delivery member 120 by forming a unitary body assembly including the flexible members 142a, 142b, and 142c and the delivery member 120. The unitary body is manufactured by, e.g., heating a plastic delivery member 120 until the plastic softens sufficiently to allow first ends 204a, 204b, and 204c (not shown for clarity of illustration) of the flexible members 142a, 142b, and 142c, respectively, to be inserted into the plastic. The flexible members 142a, 142b, and 142c attach securely to the delivery member 120 after the plastic cools. Second ends 202a, 202b, and 202c (generally 202) of the exemplary three flexible members 142a, 142b, and 142c, respectively, are free (not connected to the delivery member 120). By way of example, the flexible members 142 are manufactured using nickel-titanium material, such as Nitinol^{™} (Nitinol Devices and Components, Freemont, CA), or other shape memory alloy materials. The nickel-titanium wire, when properly manufactured, exhibits elastic properties for the wire to be manipulated (e.g., bent) by an operator and then returned to, substantially, the same shape the wire possessed prior to it being manipulated.

Properties (e.g., stiffness) of nickel-titanium wire may be varied during manufacturing of a flexible elastic member, such as the flexible elastic members 142a, 142b, and 142c. An operator may, for example, decrease the stiffness of the wire at a distal end of the flexible elastic member to minimize trauma to tissue that comes into contact with the tip of the flexible elastic member. Exemplary apparatus and methods suitable for varying properties of nickel-titanium wire are disclosed, for example, in the commonly-owned, co-pending U.S. Provisional Patent Application Serial No. 60/424,086, filed on November 6, 2002, the disclosure of which is incorporated herein by reference.

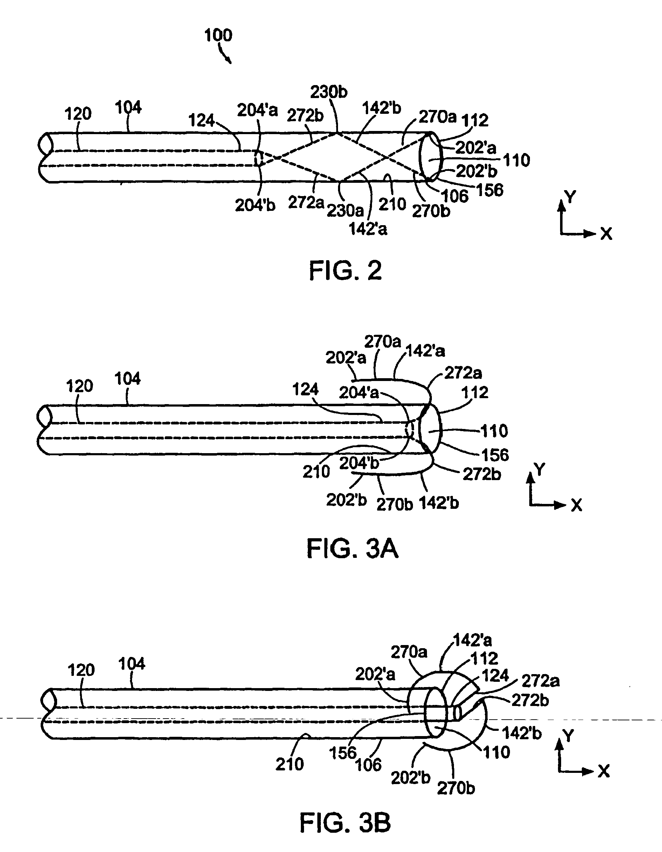

FIG. 2 illustrates a portion of a septal puncture apparatus 100 according to an illustrative embodiment of the invention including exemplary flexible members 142'a and 142'b. Each of the flexible members 142'a and 142'b include a leg such as a wire having a first end 204'a and 204'b, respectively, joined to the distal end 124 of the delivery member 120. Each of the flexible members 142'a and 142'b also have a second distal end 202'a and 202'b, respectively, that is free, i.e., not joined to any other structure of the septal puncture apparatus 100. The longitudinal axis of the flexible members 142'a and 142'b are oriented substantially parallel to the elongate member 104 when the flexible members 142'a and 142'b are located within the lumen 110 of the elongate member 104. The flexible members 142'a and 142'b have a first portion 272a and 272b, respectively and a second portion 270a and 270b, respectively. The flexible members 142'a and 142'b are disposed within the lumen 110 in this contracted position such that the second ends 202'a and 202'b are directed distally towards the opening 112 in the distal end 106 of the elongate member 104.

Prior to insertion into the lumen 110, the flexible members 142'a and 142'b are preshaped such that the flexible members 142'a and 142'b will assume a predetermined extended configuration when the flexible members 142'a and 142'b are free from the confines of the lumen 110. The flexible members 142'a and 142'b are freed from the confines of the lumen 110 by moving the flexible members 142'a and 142'b between the contracted position illustrated, for example, in FIG. 2 and an extended position, such as the extended position depicted in FIG. 3B. After insertion into the lumen 110 of the elongate member 104, the flexible members 142'a and 142'b apply a force to an inner surface 210 of the elongate member 104 in a first location 230a and 230b, respectively, on the inner surface 210 of the lumen 110 that the flexible members 142'a and 142'b contact. The force applied by the preshaped flexible members 142'a and 142'b to the inner surface 210 is the resultant force associated with the inner surface 210 constraining the shape of the flexible members 142'a and 142'b so they may fit within the lumen 110 of the elongate member 104.

In an embodiment of a septal puncture apparatus, referring now to FIG. 3A, the flexible members 142'a and 142'b are shown partially extended (in comparison with the flexible members 142'a and 142'b in FIG. 2) so the flexible members 142'a and 142'b are still substantially parallel to the longitudinal axis of the elongate body 104. As the delivery member 120 is extended out of the opening 112 of the elongate member 104, the second ends 202'a and 202'b of the flexible members 142'a and 142'b, respectively, undergo an articulation and point, generally, in a proximal direction toward the handle (not shown). In this orientation, the preshaped flexible members 142'a and 142'b apply a force to the rim 156 of the opening 112 in the elongate member 104 because the rim 156 of the opening 112 constrains the shape of the flexible members 142'a and 142'b.

The elongated delivery member 120 is further extended distally, referring now to FIG. 3B, along the lengthwise dimension (in the positive direction along the X-axis) of the lumen 110 until the distal end 124 of the delivery member 120 emerges from the opening 112 of the elongate member 104. The second ends 202'a and 202'b of the exemplary preshaped flexible members 142'a and 142'b, respectively, undergo an additional articulation and as a result point, generally, towards one another. In this extended position, the preshaped flexible members 142'a and 142'b no longer apply a force to the elongate member 104 because the elongate member 104 does not constrain the shape of the flexible members 142'a and 142'b. In this extended position, each of the flexible members 142'a and 142'b is substantially planar in shape. The plane of each of the flexible members 142'a and 142'b define a plurality of axes that lie in the plane. The plurality of axes are non-parallel to (i.e., biased relative to) the longitudinal axis of the elongate member 104. For example, the plurality of axes defined by the planes of the flexible members 142'a and 142'b are positioned at an angle in the range of about 0 degrees to about 180 degrees, preferably, about 90 degrees, relative to the longitudinal axis of the elongate member 104.

In alternative embodiments of the invention, the second ends, for example, the second ends 202'a and 202'b, may have a different diameter than other locations along the length of the flexible elastic members 142'a and 142'b. By way of example, an operator may select an apparatus having flexible members that have second ends 202'a and 202'b having a larger diameter to, for example, reduce trauma to tissue the second ends 202'a and 202'b contact during use. Alternatively, the second ends 202'a and 202'b may have a ball shaped tip.

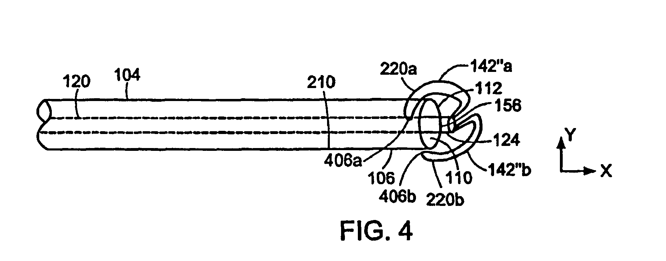

FIG. 4 depicts a portion of a septal puncture apparatus including flexible members according to an alternative illustrative embodiment of the invention. The exemplary flexible members 142"a and 142"b include a first wire loop section 220a and a second loop section 220b, respectively, as illustrated in FIG. 4. The tip 406a and 406b of the loop sections 220a and 220b, respectively, point, generally, towards one another and towards the delivery member 120. Loop sections 220a and 220b may, alternatively, be oriented in a variety of directions (e.g., away from the delivery member 120 or at a 45 degree angle away from the delivery member 120). However, the loops 220a and 220b of the flexible members 142"a and 142"b will, typically, be oriented such that the flexible members 142"a and 142"b including the loop sections 220a and 220b are substantially planar, where the plane defines a plurality of axes lying in the plane. The plurality of axes are non-parallel to (i.e., biased relative to) the longitudinal axis of the elongate member 104 when the flexible members 142"a and 142"b are extended distally from the opening 112 of the elongate member 104. Other embodiments of the flexible member 142"a and 142"b are also contemplated by the invention and are not limited to those illustrated.

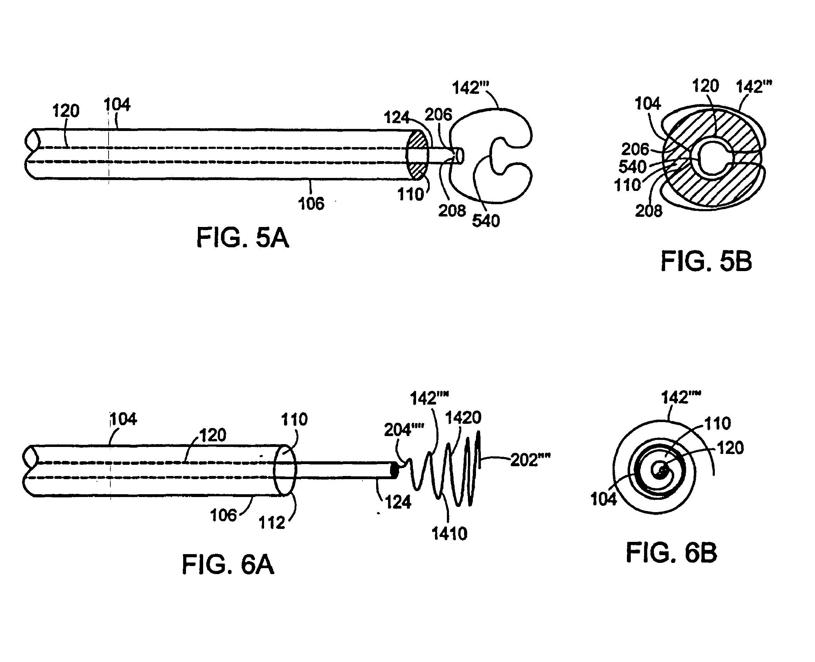

In an alternative embodiment, referring now to FIGS. 5A and 5B, septal puncture apparatus 100 includes a single flexible member 142"' that has a first end 206 and a second end 208; both the first end 206 and the second end 208 are connected to the distal end 124 of the delivery member 120. The flexible member 142"' also has a middle section 540 located, generally, intermediate the first end 206 and the second end 208 of the flexible member 142"'. The flexible member 142"' thereby forms a closed loop. In this embodiment, the flexible member 142'" is configured so the middle section 540 is located, generally in the center of a plane defined by the flexible member 142"' as illustrated by the end-on view of FIG. 5B. In this configuration, the middle section 540 of the flexible member 142"' aids with stiffening the flexible member 142"' as compared with the embodiment of the invention illustrated in FIGS. 3A and 3B where the flexible members 142'a and 142'b have free ends 202'a and 202'b, respectively. The stiffening minimizes bending when, for example, the flexible member 142"' is used by an operator to apply forces to a tissue, e.g., the atrial septum. In this configuration, the flexible member 142 forms a closed loop that is sized and shaped, for example, to contact a first and second side of a tissue, similarly, as described herein.

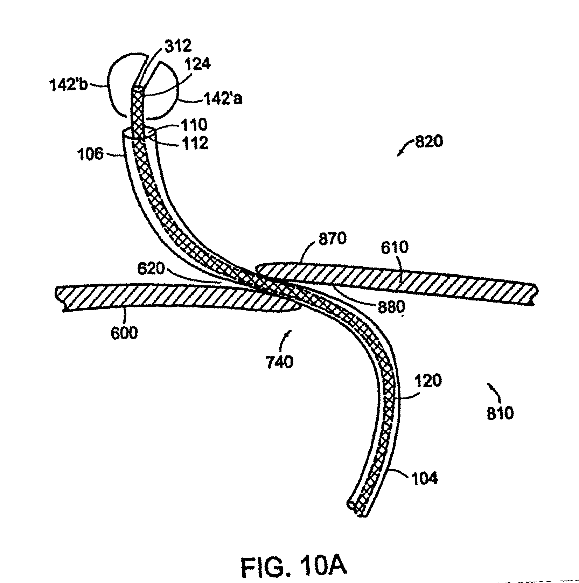

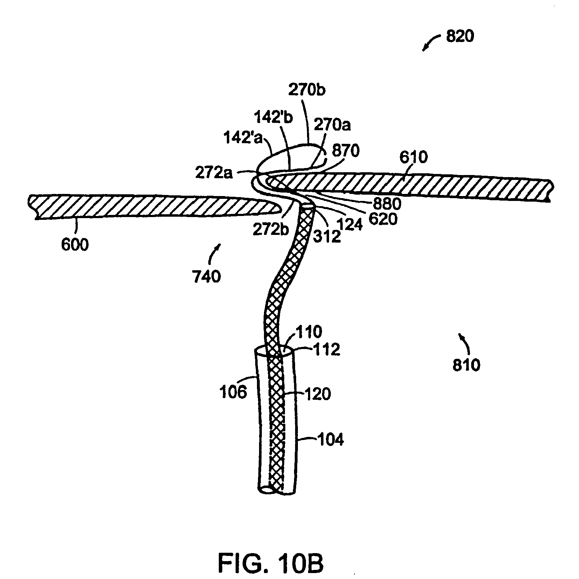

In another embodiment of the invention, referring now to FIGS. 6A and 6B, the flexible elastic member 142"" is a coil (coil-like member) and has a spiral shape extending from a narrow first end 204"" to a broad second end 202"". The narrow first end 204"" is connected to the distal end 124 of the delivery member 120. Referring now to FIG. 6B, the flexible member 142"" is oriented such that one spiral of the flexible member substantially defines a plane. The plane defines a plurality of axes lying in the plane and the plurality of axes are non-parallel to the longitudinal axis of the elongated member 104. When the delivery member 120 is withdrawn into the lumen 110 of the elongate member 104, the flexible member 142"" substantially parallels the longitudinal axis of the elongate member 104. By way of example, in use, a portion 1410 of the flexible member 142"" can be located on a first side of a tissue and a portion 1420 of the flexible member 142"" can be located on a second side of a tissue, similarly, as described herein with reference to FIGS. 10A and 10B. For example, the flexible member 142"" can be screwed through a tunnel or a hole, such as a defect in the atrial septum. Alternatively, the distal end 124 of the delivery member 120 may be located axially through, for example, a hole in a tissue such that the flexible member 142"" may be withdrawn partially through the hole by a rotational (screw-like) motion of the delivery member 120 thereby locating the portion 1410 of the flexible member 142"" on a first side of the tissue and the portion 1420 of the flexible member 142"" on a second side of a tissue.

In alternative embodiments of the spiral shaped flexible elastic member 142"", the spiral can, for example, extend from a broad first end 204"" to a narrow second end 202"", have a substantially equal diameter along the length of the spiral flexible elastic member 142"" along the longitudinal axis of the first elongate member 120, or vary in diameter along the length of the spiral flexible elastic member 142"" along the longitudinal axis of the first elongate member 120. The shape of the spiral and or parts thereof can also, for example, be chosen to approximate or match the geometry of the defect.

In one embodiment of a spiral shaped device, the flexible elastic member 142"" has the spacing between sections of the spiral that varies in relation to the longitudinal axis of the elongate member 120. By way of example, the spacing between sections of the spiral at the first end 204"" is about 1.0 mm and decreases in a linear fashion to a spacing of about 0.25 mm between sections of the spiral at the second end 202"". An operator might select the spacing between sections of the spiral that, for example, approximates the thickness of a tissue.

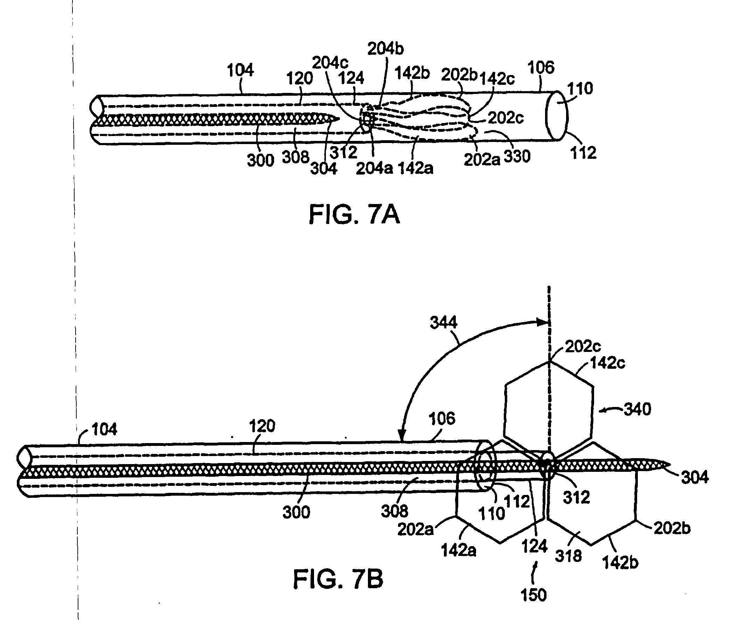

In an alternative embodiment of the present invention, as illustrated in FIGS. 7A and 7B, the cutting member 300 (e.g., a needle or sharpened wire) is slideably disposed within the lumen 308 of the delivery member 120. First ends 204a, 204b, and 204c of the exemplary three flexible members 142a, 142b, and 142c, respectively, are connected to a distal end 124 of the delivery member 120.

Referring now to FIG. 7B, the delivery member 120, the cutting member 300, and the exemplary flexible members 142a, 142b, and 142c are initially collapsed within the lumen 110 of the elongate member 104 in a contracted first position 330. The contracted flexible members 142a, 142b, and 142c are disposed within the lumen 110 of the elongate member 104 such that the flexible members 142a, 142b, and 142c lie substantially parallel to the longitudinal axis of the lumen 110 of the elongate member 104.

In this embodiment of the invention, the delivery member 120 is translated axially along the lengthwise dimension of the lumen 110 until the distal end 124 of the delivery member 120 emerges from an opening 112 in the elongate member 104 and the flexible members 142a, 142b, and 142c transition from the contracted first position 330 shown in FIG. 7A to a second extended position 340 shown in FIG. 7B. The exemplary flexible members 142a, 142b, and 142c expand to assume, for example, substantially hexagonal shapes upon emerging from the opening 112 in the elongate member 104 and expanding. The extended flexible members142a, 142b, and 142c are substantially planar. The plane defines a plurality of axes that lie in the plane and the plurality of axes are non-parallel to (i.e., biased relative to) the elongate member 104. An angle 344 defined by at least one of the plurality of axes of the plane of the flexible members 142a, 142b, and 142c and the longitudinal axis of the elongate member 104 can be between about 0 degrees and about 180 degrees. The angle 344 is typically specified (e.g., by an operator) such that the flexible members 142a, 142b, and 142c are flush with tissue surface and are capable of applying a force across a large tissue area. For example, the angle 344 might be chosen to ensure the flexible members 142a, 142b, and 142c conform to the shape of a tissue surface abutting the flexible members 142a, 142b, and 142c. If the force is applied, e.g., across a large tissue area the movement of the tissue in any location across the tissue area will be minimized. The flexible members 142a, 142b, and 142c could, alternatively, be of any shape (e.g., polygonal, circular, or ellipsoidal) or of any quantity (e.g., one, two, or five) where the shape and/or quantity of the flexible members 142a, 142b, and 142c are typically selected to distribute as much force as possible while still being able to fit within the lumen 110 of the elongate member 104 and emerge from or retract into the lumen 110.

When the flexible members 142a, 142b, 142c are extended in the second expanded position 340 upon emerging from the opening 112, the exemplary cutting member 300 extends axially in the lumen 308 of the delivery member 120 until a cutting tip 304 of the cutting member 300 emerges from the opening 312 in the distal end 124 of the delivery member 120. The tip 304 of the cutting member 300 cuts the tissue in close proximity to the opening 312 of the delivery member 120.

In an alternative embodiment, the cutting member 300 can be axially disposed in the lumen 110 of the elongate member 104 (not shown). In this alternative embodiment, the cutting member 300 is advanced until the tip 304 of the cutting member 300 emerges from the opening 112 in the elongate member 104 and travels through an opening in one of the flexible members 142 (such as opening 318 in flexible member 142b).

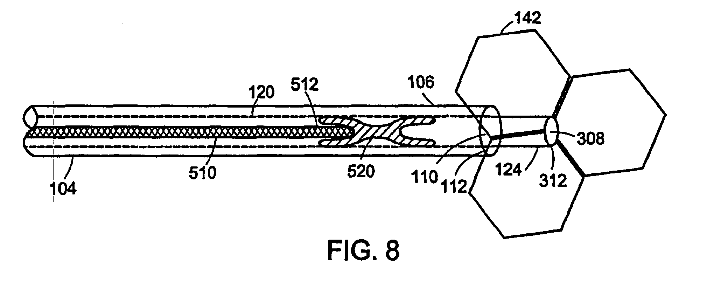

FIG. 8 depicts a portion of a septal puncture apparatus including an extending member and an occlusion device according to an illustrative embodiment of the invention. In the illustrative embodiment of the invention, an extending member 510 couples to an occlusion device, for example, an occlusion device 520, e.g., a patent foramen occluder. By way of example, occlusion devices contemplated by the invention include those as described and shown in U.S. Pat. No. 5,451,235 to Lock, et al., U.S. Pat. No. 5,578,045 to Das, and U.S. Pat. No. 6,214,029 to Thill, et al., the disclosure of each being incorporated by reference herein. The extending member 510 and the occlusion device 520 are disposed within the lumen 308 of the delivery member 120. The extending member 510 extends distally by a controller, such as the controller 138 of FIG. 1, out of the opening 312 in the distal end 124 of the delivery member 120. The occlusion device 520 may be used, e.g., to obstruct blood flow through a tissue wall in a body, such as through a patent foramen ovale in the atrial septum of the heart.

In some embodiments of the invention, a septal puncture apparatus, such as the septal puncture apparatus 100 of FIG. 1, can be used to implant alternative occlusion devices (for example, sutures, adhesives, and/or staples) into the body of the patient to join tissue. The occlusion devices may, for example, be a suture of the type described in the co-pending U.S. Provisional Patent Application "Intracardiac Suture" (NMT-022), filed simultaneously herewith, the disclosure of which is incorporated by reference. In other embodiment of the invention, a septal puncture apparatus can include a remote tissue joining apparatus (e.g., a tissue welding apparatus) that is used to join tissue in the body of the patient.

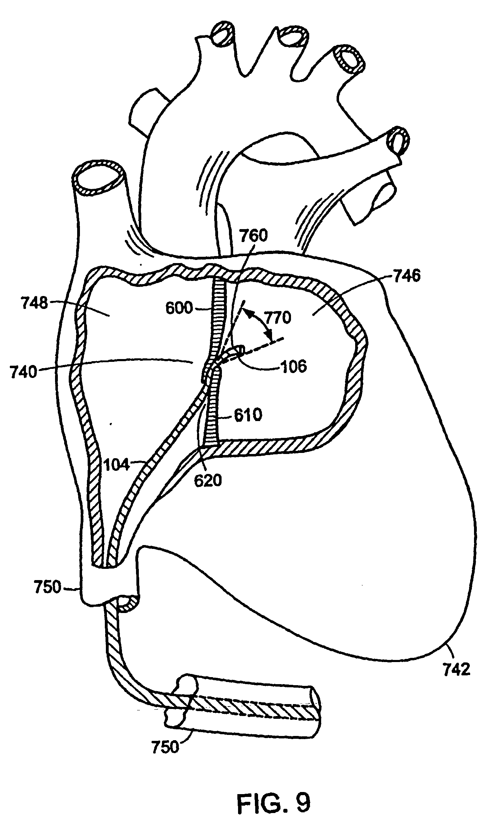

Referring now to FIG. 9, in one aspect the invention provides a method for delivery of a prosthetic occluder in the heart of a patient. According to the exemplary embodiment illustrated in FIG. 9, an operator introduces an elongate member 104 into the right atrium 748 of a heart 742 through the descending vena cava 750. The elongate member 104 could, for example, be a component of a septal puncture apparatus of the invention, such as the septal puncture apparatus 100 illustrated in FIG. 1 and described in the corresponding text.

As further illustrated in FIG. 9, the exemplary elongate member 104 is advanced distally until the distal end 106 of the elongate member 104 passes through a defect 620 (for example, a patent foramen ovale) in the septum 740. The distal end 106 of the elongate member 104 is shown at an angle 770 of about 45 degrees relative to the longitudinal axis of the elongate member 104 due to a bend 760 in the distal end of 106 of the elongate member 104. The bend 760 in the elongate member 104 may be mechanically pre-formed or pre-bent at the angle 770 between about 0 degrees and about 180 degrees prior to insertion of the elongate member into the body. The bend 760 could, alternatively, be accomplished by heating a nickel-titanium material or other shape memory alloy located within the distal end 106 of the elongate member 104. By way of example, the nickel-titanium material might be nickel-titanium wire sold under the product name Nitinol.

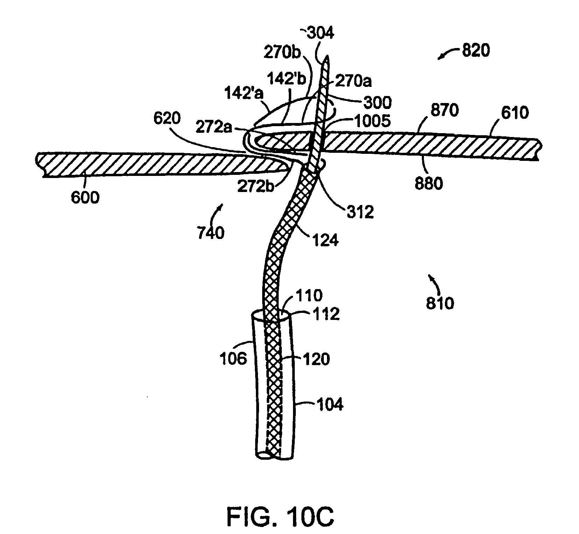

In a particular embodiment of the method for delivering an occlusion device such as a prosthetic septal occluder in the body of a patient, the illustrative septal puncture apparatus in FIGS. 10A, 10B, and 10C includes two flexible members 142'a and 142'b coupled to the distal end 124 of the delivery member 120. The flexible members 142'a and 142'b are initially located within the lumen 110 of the elongate member 104 and are in a position in which the flexible members 142'a and 142'b parallel the longitudinal axis of the elongate member 104 as previously described herein. An operator initially guides the distal end of 106 of the elongate member 104 through the defect (hole) 620 such that the distal end 106 is located on a second side 820 (in the left atria of the heart) of the septum secundum 600 and septum primum 610. Now referring to FIG. 10A, the operator then extends the flexible members 142'a and 142'b as described herein with respect to, for example, FIGS. 3A and 3B.

With continued reference to FIG. 10A, the elongate member 104 is retracted proximally until the distal end 106 of the elongate member 104 passes back through the defect 620 and is positioned on the first side 810 of the septum 740.

The delivery member 120 is then retracted proximally so the second portions 270a and 270b of the flexible members 142'a and 142'b and the distal end 124 of the delivery member 120 are in close proximity to the defect 620, the septum primum 610, and the septum secundum 600 on the second side 820 of the septum 740.

Now referring to FIG. 10B, as the delivery member 120 is further retracted proximally such that the distal end 124 of the delivery member 120 is withdrawn through the defect 620 until it is in contact with or in close proximity to the first surface 880 of the septum primum 610 on the first side 810 of the septum primum 610. The second portions 270a and 270b of the flexible members 142'a and 142'b are positioned, generally non-parallel to the longitudinal axis of the elongate member 104 and are in physical contact with at least the second surface 870 of the septum primum 610 on the second side 820 of the septum primum 610 and also partially located within the defect 620 in the septum 740. In this exemplary embodiment according to the invention, the first portions 272a and 272b of the flexible members 142'a and 142'b are located on the first side 810 of the septum 740. Accordingly, the flexible members 142'a and 142'b are sized and shaped for contact with the first side 810 and the second side 820 of the septum 740. The flexible members 142'a and 142'b are thus capable of limiting movement of the septum primum 610. Now referring to FIG. 10C, the cutting member 300 is extended from the opening 312 in the distal end 124 of the delivery member 120. The cutting tip 304 of the cutting member 300 introduces a hole 1005 (tissue opening) through the septum primum 610.

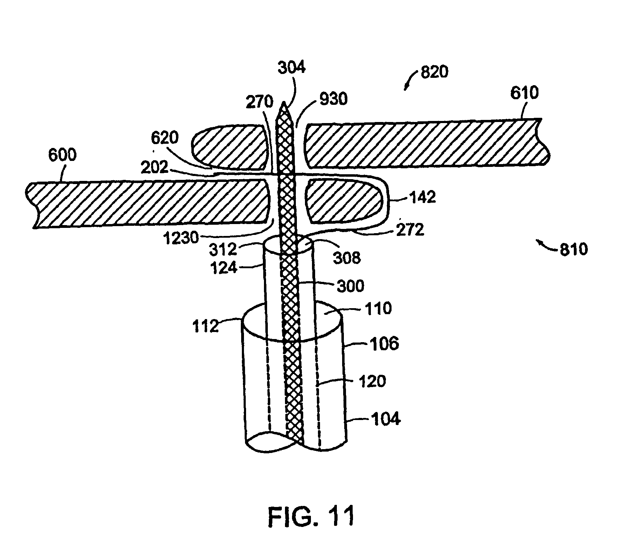

In an alternative embodiment of the invention, referring now to FIG. 11, an exemplary flexible member 142 is attached to the distal end 124 of the delivery member 120. The delivery member 120 is located within the lumen 110 of the elongate member 104. The delivery member 120 extends from the opening 112 in the distal end 106 of the elongate member 104. The delivery member 120 and the elongate member 142 are located on the first side 810 of the septum secundum 600. The distal end 124 of the delivery member 120 is located in close proximity to the tissue surface of the septum secundum 600 on the first side 810 of the septum secundum 600. The flexible member 142 extends through the hole 620 between the septum primum 610 and the septum secundum 600 from the first side 810 to the second side 820. The first side 810 of the septum primum 610 opposes the second side 820 of the septum primum 610. The flexible member 142 is positioned so that the second end 202 and second portion 270 of the flexible member 142 are located on the second side 820 of the septum secundum 600 and the first portion 272 of the flexible member 142 is located on the first side 810 of the septum secundum 600. In this configuration, the flexible member 142 is thus capable of limiting movement of the septum secundum 600. In this embodiment only the septum secundum 600 is secured to limit movement. In alternative embodiments, however, the septum secundum 600 and/or the septum primum 610 may be secured to limit movement.

In this illustrative embodiment, the cutting tip 304 of the cutting member 300 introduces a hole (tissue opening) 1230 through the septum secundum 600 and then a hole (tissue opening) 930 through the septum primum 610. For clarity of illustration purposes, the holes 1230 and 930 are shown larger in diameter than the cutting member 300.

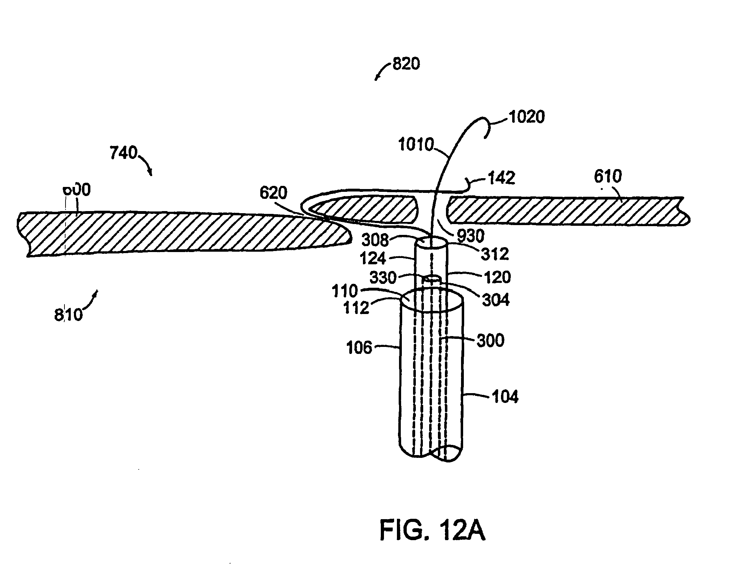

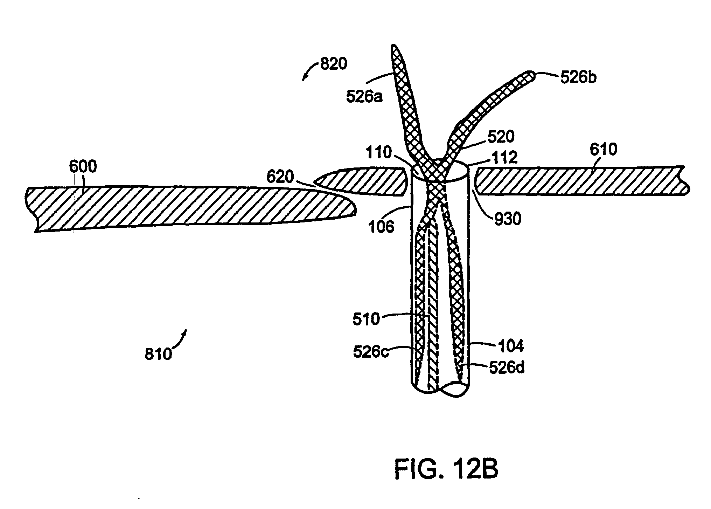

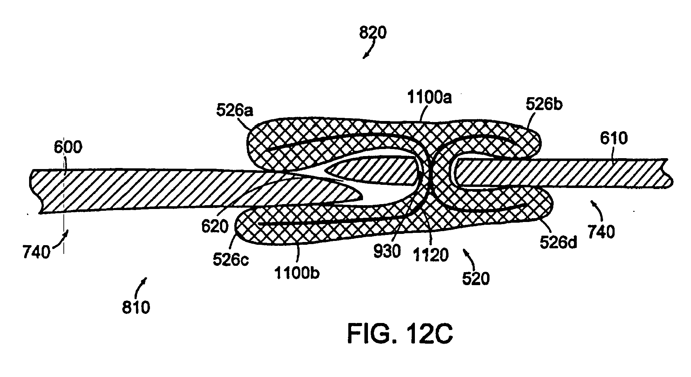

Referring now to FIGS. 12A, 12B, and 12C, the illustrative method for delivering an occlusion device, the septal puncture apparatus according to the invention includes a guidewire 1010. The illustrative guidewire 1010 is positioned by the operator within a lumen 330 of a cutting member 300. The guidewire 1010 is axially movable within the lumen 330 of the cutting member 300. The cutting member is positioned within and is axially movable within the lumen 308 of the delivery member 120. The flexible member 142 is coupled to the distal end 124 of the delivery member 120. The flexible member 142 extends from the first side 810 of the septum primum 610 through the hole 620 to the second side 820 of the septum primum 610. In this manner, the flexible member 142 limits movement of the septum primum as previously described herein.

A distal end 1020 of the guidewire 1010 extends out of the lumen 330 of the cutting member 300 and the lumen 308 of the delivery member 120 so the distal end 1020 of the guidewire 1010 is located on the second side 820 of the septum primum 610 and the septum secundum 600. By way of example, the guidewire 1010 is used by an operator to aid in guiding the elongate member 104 through the opening 930. The guidewire 1010 is then retracted proximally such that the guidewire 1010 is retracted into the lumen 110 of the elongate member and then withdrawn from the body.

Referring now to FIG. 12B, the delivery member 120 is then retracted proximally such that the flexible member 142 is retracted into the lumen 110 of the elongate member 104. The delivery member 120 is then withdrawn from the body. A dilator member (not shown) is then inserted into the lumen 110 of the elongate member 104. The dilator member is used by an operator to, for example, aid in extending the distal end 106 of the elongate member through the opening 930 in the septum primum 610 (as is known to one skilled in the art). The dilator member is then withdrawn proximally through the elongate member 104 and withdrawn from the body. The occlusion device 520 is introduced into the body by the extending member 510 through the elongate member 104. The extending member 510 is coupled to the occlusion device 520. The extending member 510 and the occlusion device 520 pass through the lumen 110 of the elongate member 104 and along the length of the elongate member 104.. The extending member 510 extends axially along the length of the lumen 110. of the elongate member 104 and the occlusion device 520 is passed distally out of the opening 112 in the elongate member 104 partially through the opening 930 in the septum primum 610. An operator manipulates the occlusion device 520 and the elongate member 104 to implant it at the anatomical site of interest so ends 526a, 526b, 526c, and 526d of the occlusion device 520 span (cover) the hole 620 and the hole 930, thereby limiting the flow of blood between the first side 810 and the second side 820 through the hole 620 and hole 930. By way of example, as the ends 526a and 526b are extended from the opening 112 of the elongate member 104 onto the second side 820 of the septum, an operator may also withdraw the elongate member 104 thereby extending the ends 526c and 526d onto the first side of the septum.

By way of example, occlusion device 520 might be a septal occluder such as the CardioSEAL® medical device manufactured by NMT Medical, Inc. with offices in Boston, MA. In accordance with the present embodiment of the invention, FIG. 12C depicts the cross-section of the occlusion device 520 that has been implanted at the anatomical site of interest (defect in the septum) to restrict blood flow through the hole 620 and the hole 930.

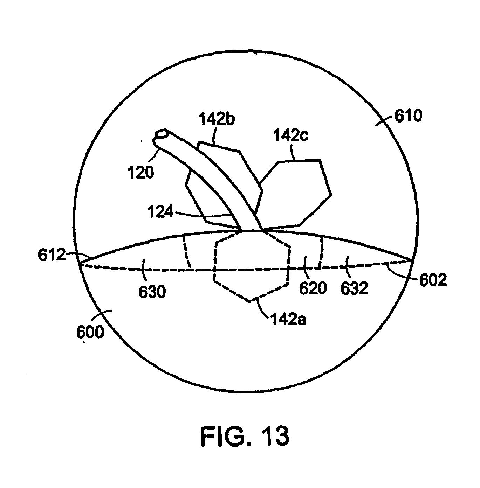

Illustrated in FIG. 13 is an alternative embodiment of the method of the invention as viewed by, for example, an operator in a side view from a first side of a septum secundum, such as a side view from the first side 810 of the septum secundum 600 of FIG. 12B. In this alternative embodiment, the illustrative septal puncture apparatus according to the invention includes the three flexible members 142a, 142b, and 142c connected to a distal end 124 of a delivery member 120. The flexible members 142a, 142b, and 142c are positioned by the operator in proximity to an opening 620 (e.g., a patent foramen ovale defect) between the septum primum 610 and the septum secundum 600. The septum primum 610 and the septum secundum 600 are partially overlapping cardiac tissue in which an edge 602 (shown with a dashed line as hidden) of the septum primum 610 overlaps with an edge 612 of the septum secundum 600. In this embodiment, the septum primum 610 and the septum secundum 600 are partially fused together in two regions 630 and 632, thereby defining the opening 620 between the septum primum 610 and the septum secundum 600.

Referring to FIG. 13, the exemplary flexible member 142a is projected through the opening 620 by the operator such that it is, at least partially, located on the side of the septum secundum 600 opposite to the side of the atrial septum on which the flexible members 142b and 142c are positioned. The flexible members 142b and 142c are located adjacent the opening 620 and sized and shaped to be in contact with an opposing side of the septum primum 610. Accordingly, in this embodiment of the invention, at least one flexible member 142 is located on a first side of a tissue, and at least one flexible member 142 is located on an opposing, second side of the tissue. In this embodiment of the invention an operator may apply forces to the septum primum 610 by pushing on the septum primum 610 with the flexible members 142b and 142c. By way of example, an operator may use this embodiment of the invention to locate the distal end 124 of the delivery member 120 in proximity to the opening 620, immobilize the septum primum 610 by pushing on the septum primum 610 with the flexible elastic members 142b and 142c, and then introduce a cutting member through a lumen of the delivery member 120 to create a hole through the septum primum 610.

Variations, modifications, and other implementations of what is described herein will occur to those of ordinary skill in the art without departing from the spirit and the scope of the invention claimed.
Embodiments of the invention may include features of the following enumerated paragraphs ("paras")
1. A surgical apparatus for forming a hole in a tissue in a patient, comprising:
   a first elongate member comprising a longitudinal axis; and
   at least one flexible member comprising a first end and a second end, the second end of said at least one flexible member free and the first end of said at least one flexible member fixed to the first elongate member, said at least one flexible member movable between a first contracted position and a second extended position, wherein in said first contracted position said at least one flexible member substantially parallels the longitudinal axis of said first elongate member, and wherein in said second extended position said at least one flexible member is substantially planar, said plane defining a plurality of axes lying in the plane, and said plurality of axes being non-parallel to said longitudinal axis of said first elongate member, wherein said at least one flexible member is sized and shaped for contact with a first side of a tissue in a patient when said at least one flexible member is in said second extended position.
2. The apparatus of para 1 wherein said at least one flexible member comprises a wire loop.
3. The apparatus of para 1 wherein said at least one flexible member comprises a section for stiffening said at least one flexible member.
4. The apparatus of para 1, wherein in said second extended position at least one of said plurality of axes defines an angle between about 0 degrees and about 180 degrees relative to the longitudinal axis of said elongate member.
5. The apparatus of para 1, wherein said at least one flexible member limits movement of the tissue when said at least one flexible member is in said second position.
6. The apparatus of para 5 further comprising a cutting member.
7. The apparatus of para 6, wherein the cutting member is axially disposed within a first lumen of the first elongate member.
8. The apparatus of para 6, wherein the cutting member comprises a needle.
9. The apparatus of para . 1 further comprising an occlusion device.
10. The apparatus of para 9, wherein the occlusion device is selected from the group consisting of a septal occluder, suture, staple, and adhesive.
11. The apparatus of para 1 further comprising an apparatus for joining tissue.
12. The apparatus of para 11, wherein the tissue joining apparatus is a tissue welding apparatus.
13. The apparatus of para 1 further comprising a second elongate member comprising a first lumen and wherein said first elongate member is for axially moving the at least one flexible member substantially co-linearly with said first lumen of said second elongate member.
14. The apparatus of para 1, wherein the plurality of axes are non-parallel to said longitudinal axis of said first elongate member by being biased relative to said first elongate member.
15. A surgical apparatus for forming a hole in a tissue in a patient, comprising:
   a first elongate member comprising at least a first lumen and a longitudinal axis; and
   a plurality of flexible members each comprising a first end and a second end, the second end of each flexible member free and the first end of each flexible member fixed relative to each other, each flexible member movable between a first contracted position and a second extended position, wherein in said first contracted position each flexible member substantially parallels the longitudinal axis of said first elongate member, and wherein in said second extended position said plurality of flexible members are substantially planar, said plane defining a plurality of axes lying in the plane, said plurality of axes being non-parallel to said longitudinal axis of said first elongate member, wherein at least one of said plurality of flexible members is in contact with at least a first surface of a tissue in a patient when said at least one flexible member is in said second extended position.
16. The apparatus of para 15, wherein at least one of said flexible members in said second extended position comprises a shape selected from the group consisting of polygonal, circular, and ellipsoidal.
17. The apparatus of para 15, wherein at least one of said plurality of flexible members is in contact with a second surface of said tissue in a patient when said flexible member is in said second extended position.
18. The apparatus of para 15 wherein at least one said plurality of flexible members comprises a wire loop.
19. The apparatus of para 15 wherein at least one of said plurality of flexible members comprises a section for stiffening the at least one flexible member.
20. The apparatus of para 15, wherein in said second extended position at least one of said plurality of axes defines an angle between about 0 degrees and about 180 degrees relative to the longitudinal axis of said elongate member.
21. The apparatus of para 15, wherein at least one of said plurality of flexible members limits movement of the tissue when the at least one flexible member is in said second position.
22. The apparatus of para 21 further comprising a cutting member.
23. The apparatus of para 22, wherein the cutting member is axially disposed within the first lumen.
24. The apparatus of para 22, wherein the cutting member comprises a needle.
25. The apparatus of para 15 further comprising an occlusion device.
26. The apparatus of para 25, wherein the occlusion device is selected from the group consisting of a septal occluder, suture, staple, and adhesive.
27. The apparatus of para 15 further comprising an apparatus for joining tissue.
28. The apparatus of para 27, wherein the tissue joining apparatus is a tissue welding apparatus.
29. The apparatus of para 15 further comprising a second elongate member coupled to at least one flexible member for axially moving the at least one flexible member substantially co-linearly with the first lumen.
30. The apparatus of para 15, wherein the plurality of axes are non-parallel to said longitudinal axis of said first elongate member by being biased relative to said first elongate member.
31. A method for stabilizing a tissue in a patient, comprising the steps of
   placing a first flexible member in contact with a first side of a tissue in a patient;
   placing a second flexible member in contact with a second side of said tissue in the patient; and
   applying pressure with at least one of said first and second flexible members to said tissue in the patient.
32. The method of para 31 further comprising the step of providing a cutting member for forming a hole in said tissue.
33. The method of para 32 further comprising the step of providing an occlusion device for occluding said hole in said tissue.
34. The method of para 33, wherein the occlusion device is selected from the group consisting of a septal occluder, suture, staple, and adhesive.
35. The method of para 32 further comprising an apparatus for joining tissue.
36. The method of para 35, wherein the tissue joining apparatus is a tissue welding apparatus.
37. A method for stabilizing a tissue in a patient, comprising the steps of
   extending a plurality of flexible members from a first lumen of a first elongate member;
   placing at least one of said plurality of flexible members in contact with at least a first surface of a tissue in a patient; and
   applying pressure with said at least one of said plurality of flexible members to said tissue in the patient.
38. The method of para 37 further comprising the step of providing a cutting member for forming a hole in said tissue.
39. The method of para 38 further comprising the step of providing an occlusion device for occluding said hole in said tissue.
40. The method of para 39, wherein the occlusion device is selected from the group consisting of a septal occluder, suture, staple, and adhesive.
41. The method of para 38 further comprising an apparatus for joining tissue.
42. The method of para 41, wherein the tissue joining apparatus is a tissue welding apparatus.
43. A surgical apparatus for producing a hole in a tissue in a patient, comprising:
   a catheter comprising a first lumen comprising an opening;
   a delivery member axially movable within the first lumen of the catheter, the delivery member comprising a first distal end extending from the catheter and a second lumen comprising an opening;
   a cutting member axially movable within the second lumen of the delivery member, the cutting member comprising a second distal end extending from the delivery member and a third lumen comprising an opening.
44. The apparatus of para 43 further comprising a guidewire axially movable within the third lumen of the cutting member, comprising a third distal end extending from the cutting member.
45. The apparatus of para 43 further comprising a flexible member comprising at least a first free end and a second free end, said at least first free end and second free end each capable of undergoing a first articulation and a second articulation.
46. The apparatus of para 45 further comprising a second elongate member coupled to the flexible member for axially moving the flexible member substantially co-linearly with the first lumen.
47. The apparatus of para 43, wherein the cutting member comprises a needle.
48. The apparatus of para 43 further comprising an occlusion device.
49. The apparatus of para 48, wherein the occlusion device is selected from the group consisting of a septal occluder, suture, staple, and adhesive.
50. The apparatus of para 43 further comprising an apparatus for joining tissue.
51. The apparatus of para 35, wherein the tissue joining apparatus is a tissue welding apparatus.
52. A surgical apparatus for producing a hole in a tissue in a patient, comprising:
   an elongate member comprising a first lumen having an opening; and
   a coil member having a first portion and a second portion and axially movable within the lumen of the elongate member, the coil member sized and shaped for being gradually transferred out of the opening in the elongate member to position said first portion of said coil member adjacent a first side of a tissue in a patient, and said second portion of said coil member adjacent a second side of said tissue in a patient.
53. The apparatus of para 52 further comprising a cutting member axially movable within the lumen of the elongate member, wherein the cutting member comprises a distal end extending from the elongate member.
54. The apparatus of para 53, wherein the cutting member comprises a needle.
55. The apparatus of para 52 further comprising a second elongate member coupled to the coil member for axially moving the at least one flexible member substantially co-linearly with the first lumen.
56. The apparatus of para 52 further comprising an occlusion device.
57. The apparatus of para 56, wherein the occlusion device is selected from the group consisting of a septal occluder, suture, staple, and adhesive.
58. The apparatus of para 52 further comprising an apparatus for joining tissue.
59. The apparatus of para 58, wherein the tissue joining apparatus is a tissue welding apparatus.

## Claims

1. A surgical apparatus for engaging an intracardiac device with a tissue in a patient, comprising:
an elongate member comprising a lumen;
a delivery member disposed within the lumen of the elongate member, said delivery member having a proximal end and a distal end; and
a flexible coil member comprising a first end and a second end, said first end being connected to the distal end of the delivery member, said flexible coil member transitionable between a first position wherein said flexible coil member is collapsed and substantially parallel to the longitudinal axis of the elongated member within the lumen of the elongate member, and a second position wherein said flexible coil member is expanded to form a spiral comprising a plurality of loops, at least one, of said loops defining a plane that is non-parallel to the longitudinal axis of the elongated member when said second portion is extended from the lumen of the elongate member.

2. The flexible coil member of claim 1, wherein the flexible coil member has a first portion and a second portion, wherein the first portion is at the first end of the coil member and is narrower than the second portion of the flexible coil member.

3. The flexible coil member of claim 1, wherein the flexible coil member has a first portion and a second portion, wherein the first portion is at the first end of the coil member and is broader than the second portion of the flexible coil member.

4. The flexible coil member of claim 1, wherein said plurality of loops have a substantially equal diameter along the length of the spiral.

5. The flexible coil member of claim 1, wherein said plurality of loops vary in diameter along the length of the spiral.

6. The apparatus of claim 1, further comprising a cutting member axially moveable within the lumen of the elongate member, wherein the cutting member comprises a distal end extending from the elongate member.

7. The apparatus of claim 6, wherein the cutting member comprises a needle.

8. The apparatus of claim 1, further comprising an occlusion device.

9. The apparatus of claim 5, wherein the occlusion device is selected from the group consisting of a septal occluder, suture, staple, and adhesive.
